Europäisches Patentamt

European Patent Office

Office européen des brevets

(11) Veröffentlichungsnummer : **0 154 163**
**B1**

(12)

# EUROPÄISCHE PATENTSCHRIFT

(45) Veröffentlichungstag der Patentschrift :
25.05.88

(51) Int. Cl.⁴ : **A 61 M  5/14**, A 61 M  5/16

(21) Anmeldenummer : 85101047.0

(22) Anmeldetag : 01.02.85

(54) Schwerkraftinfusionsregelgerät.

(30) Priorität : 07.02.84 DE 3404144

(43) Veröffentlichungstag der Anmeldung :
11.09.85 Patentblatt 85/37

(45) Bekanntmachung des Hinweises auf die Patenterteilung : 25.05.88 Patentblatt 88/21

(84) Benannte Vertragsstaaten :
AT BE CH DE FR GB IT LI NL SE

(56) Entgegenhaltungen :
DE-A- 2 906 076
GB-A- 2 118 333
US-A- 4 346 606

(73) Patentinhaber : Critikon GmbH
Mühlenweg 143
D-2000 Norderstedt (DE)

(72) Erfinder : Veracchi, F. Baldo., Dipl.-Ing.
Fuhlsbüttler Weg 83
D-2000 Hamburg 61 (DE)

(74) Vertreter : Richter, Joachim, Dipl.-Ing. et al
Patentanwälte Richter u.Werdermann Neuer Wall 10
D-2000 Hamburg 36 (DE)

## Beschreibung

Die Erfindung betrifft ein Schwerkraftinfusionsregelgerät, das aus einem Gerätegehäuse besteht, das in seinem Vorderteil eine den Konturen einer Tropfkammer entsprechend ausgebildete Ausnehmung zur Aufnahme der Tropfkammer mit einer frontal zugänglichen Einsetzöffnung für die Tropfkammer aufweist, das im Bereich der Tropfkammer eine fotoelektrische Tropfenerfassungseinrichtung aufweist und eine elektronische Einrichtung mit den Bedienungselementen sowie eine Regelmechanik aufnimmt.

Schwerkraftinfusionsregelgeräte sind in den verschiedensten Ausführungsformen im Einsatz, da sie gegenüber einer Infusionspumpe etwas kostengünstiger sind und weniger Gefahren, wie z. B. Luftembolien, ausgedehnte extravasale Infiltrationen, für den Patienten in sich bergen. Es ist allgemeine Auffassung, daß über 95 % aller Infusionen sich mit einem Schwerkraftinfusionsregelgerät bewältigen lassen würden, und daß es sehr wünschenswert wäre, wenn nahezu jedes Infusionsstativ mit einem solchen Gerät ausgestattet werden könnte, da sogar 5 %ige Glucose-Infusionslösung bei unkontrollierter Verabreichung den Patienten gefährden kann. Das erste Hindernis für die an sich erhoffte Verbreitung ist der relativ hohe Preis, der die Anwendung dieses Gerätes auf Intensivstationen oder auf hochwirksame Lösungen begrenzt. Die übliche Rollklemme, die normalerweise für die Einstellung der Tropfrate verwendet wird, kann nur als Behelfsmittel angesehen werden, und da sie keine Sicherheit bietet und häufiger Überwachungs- und Nachstellvorgänge durch das an sich sehr überlastete und teure Pflegepersonal bedarf. Der hohe Preis der gebräuchlichen Infusionsregelgeräte resultiert hauptsächlich aus der Bauweise, die im wesentlichen von der einer Infusionspumpe abgeleitet ist. An der Tropfenkammer eines derartigen Infusionsbesteckes ist ein fotoelektrischer Tropfensensor angeklemmt, der die Ist-Rate der Tropfen erfaßt. Dieser Tropfensensor ist durch ein Kabel mit dem eigentlichen Regelgerät verbunden, damit die Steuerelektronik des Gerätes selbst durch eine elektromechanische Einrichtung den Okklusionsgrad des Infusionsschlauches beeinflussen und die Ist-Tropfrate mit der eingestellten Soll-Tropfrate zur Übereinstimmung gebracht werden kann.

Die bisher bekannten Schwerkraftinfusionsregelgeräte, die mit handelsüblichen Infusionsbestecken arbeiten, sind netzbetrieben, häufig mit einem fest eingebauten wiederaufladbaren Batteriesatz versehen, der eine Notstromversorgung für drei bis vier Stunden übernehmen kann und der acht bis vierzehn Stunden Netzbetrieb zur Wideraufladung benötigt. Die so ausgebildeten Geräte sind so groß in den Abmessungen und so schwer, daß sie an ein Infusionsstativrohr angeklemmt werden müssen. Vereinzelt sind auch Geräte bekannt, die mit einer Trockenbatterie betrieben werden und damit den lästigen Netzanschluß erübrigen. Jedoch werden auch diese aufgrund der Abmessungen und des Gewichtes der Stromversorgung an Stative angeklemmt und haben den Nachteil, daß sie spezialgeartete, teure kassettenartige Sonderinfusionsbestecke benötigen, um mit einem geringen Stromverbrauch auszukommen, damit sie einer häufigen Erneuerung des Batteriesatzes mindestens teilweise entgehen können. Der teure Batteriebetrieb hat weiterhin zum Nachteil, daß die Batterien eine begrenzte Lagerzeit haben und eine ständige Lagerhaltung zwingend nötig ist.

Durch die GB-A-2 118 333 ist ein Schwerkraftinfusionsregelgerät bekannt, bei dem die Tropfkammer an der Rückseite des die elektronische Einrichtung mit den Bedienungselementen aufnehmenden Gehäuse angeordnet ist. Zur Aufnahme der Tropfkammer an dem Gerätegehäuse weist deren Rückseite eine Halterung auf. Bei diesem Infusionsschwerkraftgerät ist jedoch keine vorderseitige Einsetzöffnung für die Tropfkammer in dem Gerätegehäuse ausgebildet und auch eine Arretierung der Tropfkammer an dem Gerätegehäuse mittels einer von vorn zu bedienenden Verriegelungseinrichtung ist bei diesem Schwerkraftinfusionsregelgerät nicht vorgesehen.

Der Erfindung liegt die Aufgabe zugrunde ein leichtes, kleines und kostengünstiges, monolithisches und miniaturisiertes Schwerkraftinfusionsregelgerät zu schaffen, das nicht stativgebunden, einfach in der Handhabung und zur weiten Verbreitung bis hinunter zu peripheren Stationen geeignet ist.

Diese Aufgabe wird durch die im Patentanspruch 1 gekennzeichneten Merkmale gelöst.

Mit einem derart ausgebildeten Schwerkraftinfusionsregelgerät werden die bei den bekannten Geräten gegebenen Nachteile vermieden. Die Ausbildung und Anordnung des mit dem Infusionsschlauch zusammenwirkenden Betätigungshebels an der Vorderseite des Gerätegehäuses zur Klemmung und Halterung der Tropfkammer trägt zu einer sicheren Halterung der Tropfkammer in dem Gerätegehäuse bei und bewirkt darüber hinaus auch noch das Zusammenquetschen des Infusionsschlauches, wobei der Hebel unter der Einwirkung einer Arretierungseinrichtung steht.

Vorteilhafte Ausgestaltungen der Erfindung sind in den Unteransprüchen gekennzeichnet.

Ein Ausführungsbeispiel der Erfindung wird nachstehend anhand der Zeichnung näcfer erläutert. Es zeigt

Fig. 1 in einer Vorderansicht das Schwerkraftinfusionsregelgerät,

Fig. 2 eine Seitenansicht des Schwerkraftinfusionsregelgerätes,

Fig. 3 einen Schnitt gemäß Linie III/III in Fig. 1,

Fig. 4 eine Ansicht von oben auf das Schwerkraftinfusionsregelgerät und

Fig. 5 eine Ansicht von unten auf einen Teil des Schwerkraftinfusionsregelgerätes.

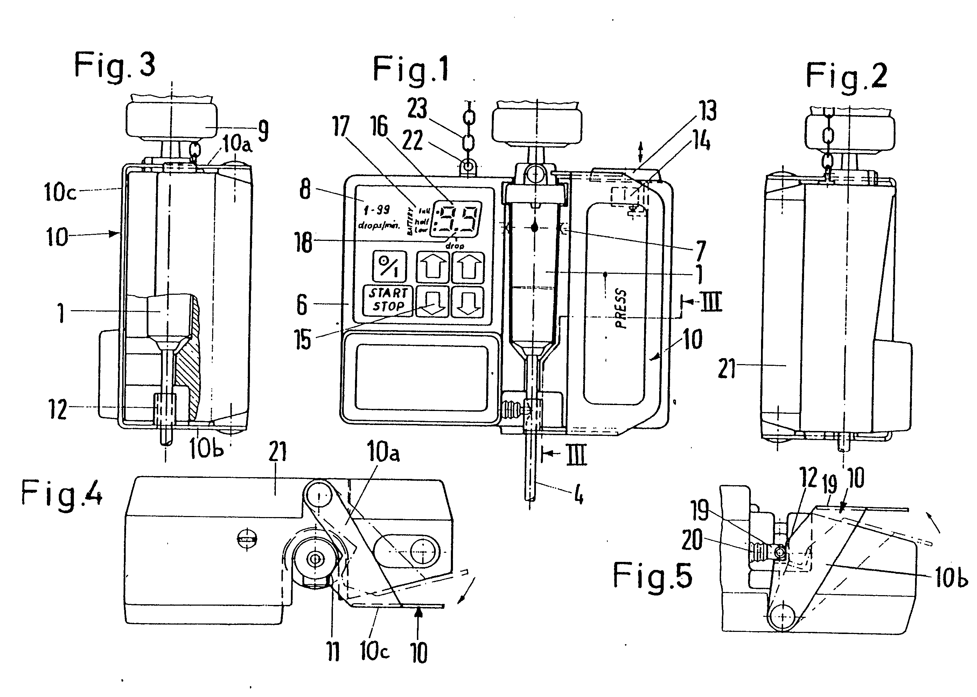

Das in den Fig. 1 bis 5 dargestellte Schwerkraftinfusionsregelgerät ist wie folgt ausgebildet :

Die bekannte zweiteilige, aus Tropfensensor und Regelgerät bestehende Bauweise ist durch einen monolithischen Aufbau ersetzt und in einem einzigen Gerätegehäuse sind eine fotoelektrische Tropfenerfassungseinrichtung 7, elektronische Einrichtungen mit den Bedienungselementen 8 und eine Regelmechanik eingeschlossen. Durch geeignete konstruktive Maßnahmen wird das Gewicht auf etwa 300 g begrenzt, damit das Gerätegehäuse direkt an die Tropfenkammer 1 angeklemmt werden kann, deren Einstichdorn so fest in dem Gummistopfen des Infusionsbehälters 9 eingeklemmt ist, daß er ohne weiteres 500 bis 600 g Zugbelastung aushalten kann.

Das Gerätegehäuse 6 weist in seinem Vorderteil eine den Konturen der Tropfkammer 1 entsprechend ausgebildete Ausnehmung zur Aufnahme der Tropfkammer 1 auf, wobei die Tropfkammer 1 frontal einsetzbar ist.

An dem Gerätegehäuse 6 ist ein U-förmiger unter der Einwirkung einer Feder stehender Hebel 10 angeordnet, dessen beide Schenkel 10a, 10b obenseitig und untenseitig an dem Gerätegehäuse 6 verschwenkbar gelagert sind und dessen die beiden Schenkel 10a, 10b miteinander verbindender Steg 10c an der Vorderseite des Gerätes als Handhabe liegend ist, wobei der obere Schenkel 10a des Hebels 10 in einer Ausnehmung 11 mit einer der Tropfkammer entsprechenden Formgebung zur Klemmung und Halterung der Tropfkammer 1 versehen ist, während der untere Schenkel 10b des Hebels 10 ein mittels des Hebels verschwenkbares, zur Anlage an dem eingelegten Infusionsschlauch 4 bringbares Widerlager 12 zum ungehinderten Einlegen des mit der Tropfkammer 1 verbundenen Infusionsschlauches 4 bei gedrückter Hebelstellung und geöffnetem Widerlager und für die Quetschung des Infusionsschlauches 4 durch ein Regelschwert 19 bei Normal-Hebelstellung aufweist, wobei der Hebel 10 gegen ein unbeabsichtigtes Betätigen mit einer knopfartigen, an dem oberen Hebelschenkel 10a anliegenden Sperre 13 in Wirkverbindung steht, die vor dem Verschwenken des Hebels 10 betätigbar ist. Damit der Hebel 10 nicht unbeabsichtigt betätigt werden kann, mit der wahrscheinlichen Folge eines Freiflusses muß erst die knopfartige Sperre 13 gedrückt werden, bevor der Hebel 10 geschwenkt werden kann.

Diese Sperre 13 dient gleichzeitig durch Betätigung eines Mikroschalters 14 als Meldeelement für die richtige Lage des Hebels 10 und konsequenterweise auch für die korrekte Montage der Tropfkammer 1 und des Infusionsschlauches 4, damit die elektronische Einrichtung im Fall von Bedienungsfehlern Alarm melden kann.

Das an dem Gerätegehäuse 6 vorgesehene Tastenfeld 15 ist aus Platzgründen vereinfacht ausgeführt und seine Arbeitsweise ist wie die eines durch Drucktasten zu betätigenden Dekodierschalters. Eine LCD-Anzeige 16 der Tropfrate, die ständig oder mit verschiedenen Frequenzen leuchtet, enthält auch die Batterieladezustandsanzeige 17 und meldet durch Blinken des Dezimalpunktes 18 das Fallen jedes Tropfens.

Die fotoelektrische Tropfenerfassung 7, bestehend aus einer Leuchtdiode (LED) und einem Fototransistor, ist in das Gerätegehäuse 6 integriert, sowie das durch einen Minischrittmotor angetriebene Getriebe so ausgelegt, daß die jeweils erforderlichen Sicherungsanforderungen erfüllt sind, wobei durch eine Nocke das Regelschwert 19 betätigt wird, um den Okklusionsgrad variieren zu können. Das Gerät ist durch geeignete Maßnahmen, wie ein Balg 20 und andere geeignete Dichtelemente gegen Schwallwasser geschützt und somit leicht zu reinigen, sowie gegen eventuelles Auslaufen der Infusionslösung.

Mit 21 ist ein Batteriekasten bezeichnet, der NiCd-Elemente enthält, der zwecks leichter Auswechselbarkeit mittels Permanent-Magneten mit relativen Gegenpolen in den vorgesehenen Gehäuseaussparungen gehalten wird, und der so bemessen ist, daß mindestens 24 Stunden Batteriebetrieb gewährleistet ist. Ein zweiter Batteriekasten wird während dieser Zeit in einem separaten Ladegerät in etwa 14 Stunden vollgeladen, so daß ein ununterbrochener Betrieb mit ausreichender Sicherheitsmarge gewährleistet ist. Durch geeignete Auswahl der elektronischen Bauteile und der Anzeigelemente ist die Schaltung so ausgelegt, daß der Stromverbrauch minimiert wird. In einer Öse 22 ist eine Kette 23 angebracht, die am Flaschenhalter befestigt werden kann, um ein versehentliches Herunterfallen des Gerätes zu vermeiden.

## Patentansprüche

1. Schwerkraftinfusionsregelgerät, das aus einem

a) Gerätegehäuse (6) besteht, das in seinem Vorderteil eine den Konturen einer Tropfkammer (1) entsprechend ausgebildete Ausnehmung zur Aufnahme der Tropfkammer (1) mit einer frontal zugänglichen Einsetzöffnung für die Tropfkammer aufweist,

b) das im Bereich der Tropfkammer (1) eine fotoelektrische Tropfenerfassungseinrichtung (7) aufweist und eine elektronische Einrichtung mit den Bedienungselementen (8) sowie eine Regelmechanik aufnimmt, dadurch gekennzeichnet,

c) daß an dem Gerätegehäuse (6) ein U-förmiger, unter der Einwirkung einer Feder stehender Hebel (10) angeordnet ist, dessen beide Schenkel (10a, 10b) obenseitig und untenseitig an dem Gerätegehäuse (6) verschwenkbar gelagert sind und dessen die beiden Schenkel (10a, 10b) miteinander verbindender Steg (10c) an der Vorderseite des Gerätegehäuses als Handhabe liegend ist,

d) daß der obere Schenkel (10a) des Hebels (10) mit einer Ausnehmung (11) mit einer der Tropfkammer entsprechenden Formgebung zur Klemmung und Halterung der Tropfkammer versehen ist und der untere Schenkel (10b) des Hebels (10) ein mittels des Hebels verschwenkba-

res, zur Anlage an dem eingelegten Infusionsschlauch bringbares Widerlager (12) zum ungehinderten Einlegen des mit der Tropfkammer verbundenen Infusionsschlauches (4) bei ge-drückter Hebelstellung und geöffnetem Widerla-ger und für die Quetschung des Infusionsschlauches (4) durch ein Regelschwert (19) bei Normal-Hebelstellung aufweist, und daß der Hebel (10) gegen ein unbeabsichtigtes Betätigen mit einer knopfartigen, an dem oberen Hebelschenkel (10a) anliegenden Sperre (13) in Wirkverbindung steht, die vor dem Verschwenken des Hebels (10) betätigbar ist.

2. Schwerkraftinfusionsregelgerät nach Anspruch 1, dadurch gekennzeichnet, daß die Sperre (13) in Verbindung mit einem Mikroschalter (14) als Meldeeinrichtung zur Anzeige der richtigen Position des Hebels (10) und des funktionsgerechten Einsatzes der Tropfkammer (1) in der Ausnehmung in dem Gerätegehäuse (6) steht, wobei bei fehlerhaftem Einsetzen der Tropfkammer und fehlerhaftem Anbringen des Infusionsschlauches (4) eine Warneinrichtung betätigt wird.

3. Schwerkraftinfusionsregelgerät nach Anspruch 1 und 2, dadurch gekennzeichnet, daß in dem Gerätegehäuse (6) ein Batteriekasten (21) angeordnet ist, der vermittels Permanent-Magneten in einer in dem Gehäuse (6) ausgebildeten und der Form des Batteriekastens (21) entsprechenden Ausnehmung gehalten ist.

4. Schwerkraftinfusionsregelgerät nach einem der Ansprüche bis 3, dadurch gekennzeichnet, daß das Gerätegehäuse (6) mittels einer am Flaschenhalter für den Infusionslösungsbehälter zu befestigenden Sicherheitskette (23) versehen ist.

**Claims**

1. Gravity infusion control device consisting of
a) a case (6) with a recess in its front part that matches the contour of a drip chamber (1) for reception of the drip chamber (1) with an introduction opening to allow access from the front for the drip chamber,
b) which has a photoelectric drip monitoring device (7) in the area of the drip chamber (1) and receives an electronic device with the control elements (8) and a control mechanism, characterized in
c) that a U-shaped, spring-controlled lever (10) is positioned on the case (6), the two legs (10a, 10b) of which are hinged with their upper and lower sides to the case (6) so that they can be pivoted and the bridge (10c) of which linking the two legs (10a, 10b) is arranged on the front of the case in the form of a handle,
d) that the upper leg (10a) of the lever (10) has a recess (11) of a shape that corresponds to the drip chamber for clamping and holding the drip chamber and the lower leg (10b) of the lever (10) has a backing bearing (12) that can be pivoted by means of the lever for approach to the introduced infusion tube allowing unhindered introduction of the infusion tube (4) which is connected with the drip chamber when the lever is pressed down and the backing bearing is open and allowing the squeezing of the infusion tube (4) by means of a control sword (19) with the lever in normal position, and that the lever (10) is in contact with a knob-like lock (13) lying on the upper leg of the lever (10a) to prevent unintentional actuation, the lock being such that it can be actuated prior to the pivoting of the lever (10).

2. Gravity infusion control device according to claim 1, characterized in that the lock (13) is connected to a microswitch (14) as an indication device for the display of the correct position of the lever (10) and the functional introduction of the drip chamber (1) in the recess in the case (6), whereby an alert system is generated in the event of faulty introduction of the drip chamber and incorrect application of the infusion tube (4).

3. Gravity infusion control device according to claims 1 and 2, characterized in that a battery chamber (21) is provided in the case (6), which, by means of permanent magnets, is held in the case (6) in a recess that matches the form of the battery chamber (21).

4. Gravity infusion control device according to claims 1-3, characterized in that the case (6) is provided with a safety chain (23) that is to be fixed to the bottle holder for the container of the infusion solution.

**Revendications**

1. Appareil de réglage de perfusion par gravité qui se compose
a) d'un boîtier d'appareil (6) qui présente, dans sa partie antérieure, un creux formé de manière à correspondre aux contours d'une chambre d'égouttement (1) pour loger la chambre d'égouttement (1) avec un orifice de mise en place de la chambre d'égouttement accessible frontalement,
b) qui présente, dans la zone de la chambre d'égouttement (1), un dispositif photoélectrique de détection des gouttes (7) et qui loge un dispositif électronique avec les éléments de commande (8) ainsi qu'un mécanisme de réglage, caractérisé en ce
c) qu'un levier en forme de U (10) se trouvant sous l'influence d'un ressort est placé sur le boîtier d'appareil (6), levier dont les deux montants (10a, 10b) sont positionnés de manière à être pivotants du côté supérieur et du côté inférieur du bâti d'appareil (6) et dont le dos (10c), qui relie les deux montants (10a, 10b) l'un à l'autre, est situé sur la face antérieure du bâti d'appareil comme poignée,
d) que le montant supérieur (10a) du levier (10) est pourvu d'un creux (11) ayant une configuration correspondante à celle de la chambre d'égouttement pour le blocage et le maintien de la chambre d'égouttement et que le montant inférieur (10b) du levier présente une butée (12), pivotante au moyen du levier, qui peut être amenée contre le tuyau de perfusion mis en place, pour la mise en place sans entraves du tuyau de perfusion (4) relié à la chambre d'égouttement, lorsque le levier est en position enfoncée et que la butée est ouverte, et pour le pincement du tuyau de perfusion (4) dans une semelle de réglage (19) en position normale du levier, et que le levier (10) est en relation active avec un arrêt (13) du type bouton, adhérent au montant supérieur du levier (10a), contre un actionnement inopportun, arrêt qui peut être actionné avant de faire pivoter le levier (10).

2. Appareil de réglage de perfusion par gravité selon la revendication 1, caractérisé en ce que l'arrêt (13) est en relation avec un microrupteur (14) comme dispositif de signalisation pour l'indication de la position juste du levier (10) et de la mise en place appropriée au fonctionnement de la chambre d'égouttement (1) dans le creux du bâti d'appareil (6), un dispositif d'alarme étant actionné en cas de mise en place incorrecte de la chambre d'égouttement et du placement incorrect du tuyau de perfusion (4).

3. Appareil de réglage de perfusion par gravité selon les revendications 1 et 2, caractérisé en ce qu'une caisse de batterie (21) est placée dans le boîtier d'appareil (6), caisse de batterie qui est maintenue au moyen d'aimants permanents dans un creux formé dans le boîtier (6) et correspondant à la forme de la caisse de batterie (21).

4. Appareil de réglage de perfusion par gravité selon l'une des revendications 1 à 3, caractérisé en ce que le boîtier d'appareil (6) est pourvu d'une chaîne de sécurité (23) qui doit être fixée au porte-flacon du récipient de la substance de perfusion.

Fig. 3

9
10a
10c
10
1
12
10b

Fig. 1

23
17 16 22
8
1-99
drops/min.
BATTERY
full
half
low
18
drop
6
%/1
START
STOP
15
13
14
7
1
PRESS
III
III
10
4

Fig. 4

21
10a
11 10c 10

Fig. 2

13
14
21

0 154 163

Fig. 5

12 19 10
19
20
10b